# EUROPEAN PATENT APPLICATION

(11) **EP 2 500 028 A1**
(43) Date of publication of application: **19.09.2012**
(21) Application number: 10830082.3
(22) Date of filing: 20.04.2010
(51) Int. Cl.: A61K 36/81, A61P 1/16, A61K 36/23, A23L 1/30

(54) **COMPOSITION FOR ALLEVIATING FATTY LIVER**

(30) Priority: 11.11.2009 KR 20090108694
(71) Applicant: Nam, Jong Hyun, Songpa-gu, Seoul 138-110 (KR)
(72) Inventor: Nam, Jong Hyun, Songpa-gu, Seoul 138-110 (KR)
(74) Representative: Isarpatent
(86) International application number: PCT/KR2010/002460
(87) International publication number: WO 2011/059149

(57) **Abstract**

The present invention relates to a composition for improving fatty liver, including at least one selected from the group consisting of a whole tomato plant extract, a Torilis japonica decandolle extract and a Lithospermum erythrorhizon Siebold & Zucc extract as an active ingredient.

## Description

### Technical Field

The present invention relates to a composition for improving fatty liver. More particularly, the present invention relates to a composition including an extract from a whole tomato plant and/or other herbs as an active ingredient improving fatty acid.

### Background Art

Fatty liver is a condition where large vacuoles of triglyceride fat accumulate in liver cells via the process of steatosis associated with an abnormal fat metabolism.

Clinically, the diagnosis of fatty liver is made when fat is observed in more than 5% of hepatocytes or when fat in the liver exceeds 5 % by weight (i.e., 5 mg per 100 mg of liver). Recently, there has been an increase in fatty liver patients due to high-fat, high-calorie diets and insufficient exercise, with the onset thereof over a wide range of ages from the teens to the fifties and sixties. Without suitable treatment, fatty liver may develop into hepatitis, hepatocirrhosis and liver cancer.

Fatty liver is commonly associated with alcohol, diabetes, obesity, malnutrition (a low protein diet for a long period of time, a high-fat diet, starvation, insufficient vitamins, excessive glucose uptake, etc.) and drug abuse (KIM S.H. et al., "Causes of fatty liver diagnosed by abdominal ultrasonography" J. Korean Acad. Fam. Med. 16(11), pp.785-794 (Nov. 1995))

Fatty liver is further classified into cholesterol fatty liver, phospholipid fatty liver and triglyceride fatty liver. Most common is triglyceride fatty liver, which is caused by the abnormal accumulation of triglycerides in the liver.

Among commercially available therapeutics for fatty liver are Legaron™ (BukwngPharm Co. Ltd), Recover™ (Yuhan), Heparegen™ (Boryung Pharm), and Hepa-Merz™ (Hanwha Pharm). Korean publicized Patent No. 2006-0028561 discloses the use of genestein in improving fatty liver.

### Disclosure

### Technical Problem

It is an object of the present invention to provide a composition for the improvement of fatty liver, including an extract from a whole tomato plant and/or other herbs.

Other technical features will be apparent from the following description.

### Technical Solution

The present invention addresses a composition for improving fatty liver, including an extract from a whole tomato plant and/or other plants as an active ingredient.

Leading to the present invention, intensive and thorough research into the improvement of fatty liver, conducted by the present inventor, resulted in the finding that extracts of a whole tomato plant, Torilis japonica decandolle and Lithospermum erythrorhizon Siebold & Zucc or a mixture of 50% ethanol extracts thereof has the activity of decreasing blood glutamate- pyruvate transaminase (hereinafter referred to as "GPT") and glutamate-oxaloacetate transaminase (hereinafter referred to as "GOT") levels and total liver cholesterol and triglyceride levels in animals fed with a high-fat diet or alcohol. GPT and GOT are enzymes that are released from the liver into the blood when the liver is damaged by alcohol or obesity and thus can be used to indicate liver dysfunction. It is known that a substance capable of decreasing GPT and GOT activities can improve liver function (M. Hayashi, et al., Nippon Yakuri gaku Zasshi, 100, 391-399(1992)).

Based on the results of the tests, the present invention provides a composition for improving fatty liver, including at least one selected from the group consisting of a whole tomato plant extract, a Torilis japonica decandolle extract and a Lithospermum erythrorhizon Siebold & Zucc extract (hereinafter referred to as "whole tomato plant extract and the like") in accordance with an aspect thereof.

As used herein, the term "fatty liver" is intended to include the above-mentioned three types of fatty liver, i.e., cholesterol fatty liver, phospholipid fatty liver and triglyceride fatty liver, irrespective of its cause, i.e. alcoholic or non-alcoholic fatty liver. A fatty liver, whether induced by alcohol or a non-alcoholic cause, accumulates cholesterol or triglycerides therein. As will be elucidated later, the whole tomato plant extract and the like exhibit the activity of reducing total liver cholesterol and triglyceride levels in vivo.

As used herein, the term "improvement" is intended to mean the prevention, amelioration, treatment or delay of a symptom associated with an illness.

The term "whole tomato plant extract," as used herein, is intended to include not only an extract from any one part of a tomato plant, such as the leaves, stems, flowers, roots or a combination thereof, using a solvent such as methanol, ethanol, acetone, ethylacetate, saturated n-butanol, chloroform, methylene chloride, water or a combination thereof, irrespective of extraction methods, but also a fraction of the extract in such a solvent. So long as it includes the immersion of the extraction target, such as tomato leaves, stems, flowers, roots or a combination thereof, in a solvent, any extraction method may be used in the present invention. Examples of the extraction method include cold precipitation, refluxing, warming, and ultrasonication. Preferably, the "whole tomato plant extract" is obtained by immersing the extraction target selected from the group consisting of tomato leaves, stems, flowers, roots and combinations thereof in a solvent selected from the group consisting of water, ethanol or a combination thereof to give an extract of a concentrated liquid phase or a solid phase from which the solvent is removed.

The term *" Torilis japonica decandolle* extract" is defined in the same manner as was "whole tomato plant extract," with the exception that the extraction target includes *Torilis japonica decandolle* leaves, stems, flowers, roots and a combination thereof.

Likewise, the term *"Lithospermum erythrorhizon* Siebold & Zucc extract" is defined in the same manner as was "whole tomato plant extract," with the exception that the extraction target includes *Lithospermum erythrorhizon* Siebold & Zucc leaves, stems, flowers, roots and a combination thereof.

Other terms that are not defined separately herein adhere to the meanings found in dictionaries or generally accepted in the art.

Any amount of the active ingredient such as a whole tomato plant extract and the like may be contained in the composition for improving fatty liver in accordance with the present invention so long as it effectively works to improve fatty liver. The effective amount of the active ingredient varies depending on the use, formula, and formulation purpose of the composition, and is generally on the order of 0.001 % by weight to 99.900 % by weight based on the total weight of the composition. By the term "effective amount" is meant the amount of the active ingredient sufficient to result in inducing an improvement in a fatty liver. Determining the amount experimentally is within the ordinary ability of a person skilled in the art.

Without suitable treatment, fatty liver is likely to progress to hepatitis, hepatocirrhosis and liver cancer.

Accordingly, the whole tomato plant extract and the like may be useful for the prevention of hepatitis, hepatocirrhosis and liver cancer which are developed from fatty liver.

In accordance with another aspect thereof, therefore, the present invention provides a composition for the prevention of a disease associated with fatty liver, including a whole tomato plant extract and the like as an active ingredient.

A description given to the composition for the improvement of fatty liver with regard to preferred embodiments of the active ingredient and its effective amount is true of the composition for the prevention of a disease associated with fatty liver.

In accordance with a further aspect thereof, the present invention provides a composition for the mitigation of hangover, including a whole tomato plant extract and the like as an active ingredient.

Hangover is the experience of various unpleasant physiological effects, such as nausea and headache, following the heavy consumption of alcoholic beverages and is contributed to by products formed by the breakdown of ethanol by liver enzymes, primarily by aldehyde. In the liver, alcohol dehydrogenase (ADH) converts alcohol into acetaldehyde which is in turn oxygenized to acetate, a non-toxic substance, by aldehyde dehydrogenase (ALDH).

As will be elucidated in the following Example section, the whole tomato plant extract and the like can be used to mitigate a hangover because they promote ADH and ALDH activity.

Preferably, the composition for the mitigation of hangover in accordance with the present invention may further include a combination of a whole tomato plant extract, a Sorbus commixta extract and an Alnus japonica Steud as an active ingredient. This is because, as proven in the following Example section, a combination of a whole tomato plant extract, a Sorbus commixta extract and an Alnus japonica Steud remarkably increases ADH and ALDH activity.

The Sorbus commixta extract and the Alnus japonica Steud extract are defined in the same manner as was "whole tomato plant extract," with the exception that their extraction targets include Sorbus commixta and Alnus japonica Steud leaves, stems, flowers, roots and a combination thereof, respectively.

The description given of the composition for the improvement of fatty liver with regard to preferred embodiments of the active ingredient and its effective amount is also true of the composition for the mitigation of hangover.

Contemplated in accordance with still a further aspect of the present invention is a composition for the improvement of obesity, including a whole tomato plant extract and the like as an active ingredient.

Obesity is a metabolic disease resulting from the imbalance between calorie intake and consumption and refers to a medical condition in which adipose tissues are abnormally increased due to excessive caloric intake [Kopelman PG. Neuroendocrine function in obesity. Clinical Endocrinology 28:675-689, 1998; Clinical Obesity (eds. Kopelman PG, Stock MJ) 248-89 (Blackwell Science, Oxford 1998)]. People are diagnosed with obesity when the body fat exceeds 25% of the body weight for men and 30% of the body weight for women. The body mass index (BMI) defines people as overweight if their BMI is between 25 and 30, and obese when it is greater than 30.

At an individual level, a combination of environmental factors including excessive food energy intake, a lack of physical activity and endocrinal disorders are thought to explain most cases of obesity. A limited number of cases are due primarily to genetics, medical reasons, or psychiatric illness. The environmental factors are responsible for 50∼70% of the cases of obesity while genetic and other factors account for the remainder.

As used herein, the term "obesity" is intended to refer to a condition in which adipose tissues abnormally increase whether because of environmental factors or genetic factors and includes both obesity and being overweight as defined by a body mass index of 30.0 and 25∼30, respectively.

The description given for the composition for the improvement of fatty liver with regard to preferred embodiments of the active ingredient and its effective amount is also true of the composition for the improvement of obesity.

When maintained for a long period of time, an obese condition may act as a cause of various diseases of which metabolic diseases such as hypertension and diabetes are representative (Kim Chang Jong, Obesity and Health, 2002, Chung Ang University Press).

Therefore, in accordance with still another aspect thereof, the present invention provides a composition for the prevention of a disease associated with obesity, including a whole tomato plant extract and the like as an active ingredient.

In accordance with still yet another aspect thereof, the present invention provides a diet composition including a whole tomato plant extract and the like as an active ingredient.

As used herein, the term "diet" is intended to refer to a condition in which a reduction in the body weight/body fat is desired or necessary for the purpose of aesthetics or health although unrelated to either being overweight or obese. Generally, the diet composition of the present invention may be prepared for normal people for the sake of aesthetics or health.

The description given of the composition for the improvement of fatty liver with regard to preferred embodiments of the active ingredient and its effective amount is also true of the diet composition of the present invention.

According to another embodiment, the compositions for improving fatty liver, for preventing a disease associated with fatty liver, for mitigating an overhang, for improving obesity, for preventing a disease associated with obesity, and for dieting (hereinafter referred to as "the compositions of the present invention") may be food compositions for, for example, beverages.

The food composition may contain an additive such as a sweetener, a flavoring agent, a physiologically active substance, a mineral, etc. in addition to the active ingredient.

A sweetener is used to give a sweet taste to the composition and may be natural or synthetic. Preferable is a natural sweetener. Examples of the natural sweetener include corn syrup, honey, sucrose, fructose, lactose, maltose and other sugars.

A flavoring agent is adopted to enhance the taste or flavor of the composition and may be natural or synthetic. Preferable is a natural flavoring agent. A flavoring agent, if natural, may have the function of nutritional supplementation in addition to enhancing the flavor. Examples of the natural flavoring agent include those obtained from apple, lemon, mandarin, grape, strawberry, peach, green tea leaves, Polygonatum odoratum, bamboo leaves, cinnamon, chrysanthemum leaves, and/or jasmine. Other natural flavoring agents include those from ginseng (red ginseng), bamboo shoots, aloe vera, and/or ginkgo nuts. The natural flavoring agent may be in the form of a liquid concentrate or a solid extract. A synthetic flavoring agent may be used, and is exemplified by esters, alcohols, aldehydes and terpenes.

Among the physiological active substance are catechins, such as catechin, epicatechin, gallocatechin and epigallocatechin, and vitamins, such as retinols, ascorbic acid, tocopherol, calciferol, thiamine and rivoflavin.

As for the mineral, examples thereof include calcium, magnesium, chrome, cobalt, copper, fluorides, germanium, iodine, iron, lithium, magnesium, manganese, molybdenum, phosphorus, calcium, selenium, silicone, sodium, sulfur, vanadium, and zinc.

Optionally, the food composition of the present invention may further include a preservative, an emulsifier, an acidulant, and a thickener in addition to the additives such as a sweetener, etc.

These agents such as preservatives, emulsifiers, etc. are used in as minimal an amount as possible to achieve the purpose of their addition. Numerically, their amount ranges from approximately 0.0005 % by weight to 0.5 % by weight based on the total weight of the composition.

Examples of the preservative useful in the present invention include calcium sorbate, sodium sorbate, potassium sorbate, calcium benzoate, sodium benzoate, potassium benzoate, and EDTA (ethylenediaminetetracetic acid).

Acacia gum, carboxymethylcellulose, xanthan gum, and pectin are emulsifiers that can be used in the present invention.

Representative among the acidulants are citric acid, malic acid, fumaric acid, adipic acid, phosphoric acid, gluconic acid, tartaric acid, ascorbic acid and acetic acid. The acidulant may be added for the purpose of suppressing microbial proliferation as well as enhancing the taste.

The thickener useful in the present invention may be exemplified by a suspending agent, a flocculant, a gel forming agent, and a swelling agent.

In accordance with another embodiment, the composition of the present invention may be used as a pharmaceutical composition.

The pharmaceutical composition according to the present invention comprises a pharmaceutically acceptable carrier in addition to the active ingredient and may be formulated into oral dosage forms (tablets, suspensions, granules, emulsions, capsules, syrup, etc.), parenteral dosage forms (sterile injections, aqueous or oily suspensions, etc.), and topical application forms (solutions, creams, ointments, gels, lotions, patches, etc.).

As used herein, the term "pharmaceutically acceptable" means that a material does not interfere with the effectiveness of the biological activity of the active ingredients and is low enough in toxicity to be used on the subject.

Examples of the pharmaceutically acceptable carrier include lactose, glucose, sucrose, starch (e.g., corn starch, potato starch, etc.), cellulose and its derivatives (e.g., sodium carboxymethyl cellulose, ethyl cellulose, etc.), malt, gelatin, talc, a solid lubricant (e.g., stearic acid, magnesium stearate, etc.), calcium sulfate, vegetable oil (e.g., peanut oil, cotton seed oil, sesame oil, olive oil), polyol (e.g., propylene glycol, glycerine), alginic acid, emulsifiers (e.g., TWEENS), wetting agents (sodium lauryl sulfate), colorants, flavoring agents, stabilizers, antioxidants, preservatives, water, saline, and phosphate buffered saline. These carriers may be used, individually or in combination, according to the formulation of the pharmaceutical composition.

A suitable excipient may be also employed in the pharmaceutical composition of the present invention. For example, an excipient suitable for formulating the pharmaceutical composition of the present invention into an aqueous suspension may be a suspending agent or dispersant such as sodium carboxymethyl cellulose, methyl cellulose, hydropropylmethylcellulose, sodium alginate, or polyvinylpyrrolidone. When the pharmaceutical composition is formulated into an injection, Ringer's solution, or isotonic sodium chloride may be used as an excipient.

To administer the pharmaceutical composition of the present invention, an oral route or a parenteral route such as a topical route may be taken.

The daily dose pharmaceutically composition of the present invention may be administered at a daily dose of from 0.001 ∼ 150 mg/kg of body weight and in a single dose or in multiple doses per day. The dose of the pharmaceutical composition of the present invention may vary depending on various factors including the route of administration, the patient's age, gender and weight, and the severity of illness and thus must be in no way understood to limit the scope of the present invention.

### Advantageous Effects

As described hitherto, a composition including a whole tomato plant extract and the like is provided to improve fatty liver in accordance with the present invention. Also, the present invention provides compositions including a whole tomato plant extract and the like for the prevention of fatty liver-associated diseases such as hepatitis, hepatocirrhosis and liver cancer, for the mitigation of hangover, for the improvement of obesity, and for the prevention of obesity-associated diseases such as hypertension and diabetes. Further, the present invention provides a diet composition based on a whole tomato plant extract and the like.

The compositions may be used as food compositions for gum or beverages or as pharmaceutical compositions.

### Mode for Invention

A better understanding of the present invention may be obtained through the following examples which are set forth to illustrate, but are not to be construed as limiting the present invention.

### REFERENCE EXAMPLES: Preparation of Extracts from Alnus japonica Steud and Sorbus commixta

### REFERENCE EXAMPLE 1: Preparation of Alnus japonica Steud Extract

Leaves, stems and roots taken from Alnus japonica Steud were washed, dried and sliced. 1 kg of the sliced Alnus japonica Steud was immersed in 5ℓ of 50 % ethanol at 50°C for 10 hours. After removal of the plant debris, the solvent was removed by concentration at reduced pressure to afford an extract in a solid phase.

### REFERENCE EXAMPLE 2: Preparation of Sorbus commixta

Leaves, stems and roots taken from Sorbus commixta were washed, dried and sliced. 1 kg of the sliced Sorbus commixta was immersed in 5ℓ of 50 % ethanol at 50°C for 10 hours. After removal of the plant debris, the solvent was removed by concentration at reduced pressure to afford an extract in a solid phase.

WORKING EXAMPLES: Preparation of Extracts from Whole Tomato Plant, Lithospermum erythrorhizon Siebold & Zucc and Torilis japonica decandolle

### WORKING EXAMPLE 1: Preparation of Extract from Whole Tomato Plant

1 kg of sliced whole tomato plant was immersed in 5ℓ of 50 % ethanol at 50°C for 10 hours, followed by removal of the plant debris. The solution was concentrated at a reduced pressure to remove the solvent and to afford an extract in a solid phase.

### WORKING EXAMPLE 2: Preparation of Extract from Torilis japonica decandolle

1 kg of sliced Torilis japonica decandolle was immersed in 5ℓ of 50 % ethanol at 50°C for 10 hours, followed by removal of the plant debris. The solution was concentrated at a reduced pressure to remove the solvent and to afford an extract in a solid phase.

### WORKING EXAMPLE 3 : Preparation of Extract from Lithospermum erythrorhizon_Siebold & Zucc

1 kg of sliced Lithospermum erythrorhizon Siebold & Zucc was immersed in 5ℓ of 50 % ethanol at 50°C for 10 hours. After the removal of the plant debris, the solvent was removed by concentration at a reduced pressure to afford an extract in a solid phase.

### EXPERIMENTAL EXAMPLES: Assays for Activity of Improving Liver Function, Mitigating Hangover and Suppressing Obesity

### EXPERIMENTAL EXAMPLE 1: Assay for Activity of Improving Liver Function

### EXPERIMENTAL EXAMPLE 1-1: Improvement of Obesity-Induced Fatty Liver

### EXPERIMENTAL EXAMPLE 1-1-1: Measurement of GPT and GOT

Six-week-old, specific pathogen-free, Spraque-Dawley rats, weighing 220±8.9g on average, were purchased from Central Lab. Animal Inc. Korea. They were divided into five groups of five in respective cages and acclimated for 2 weeks to the test environment before testing and were allowed to freely approach sufficient amounts of water and food.

Of the five groups, one was used as a negative control (normal) fed with a normal diet (AIN-76 food, Feedlab), one as a positive control fed with a high-fat diet (normal diet + 30 (wt parts, 30 wt parts per 100 wt parts of normal diet) fat mix), and the other three groups as test groups fed with the high-fat diet plus the extract (20 mg/kg). The feeding continued for six weeks. They were bred in an animal breeding room automatically maintained at a constant temperature (21±2°C) and a relative humidity (60±5%) with a light/dark cycle of 12L/12D, with free access to water and the diet permitted thereto.

After the breeding was completed, the activity of the enzymes indicative of liver damage was examined in the rats. Blood samples (2 mℓ) were taken from the abdominal vena cava of the rats and centrifuged at 4500 × g for 20 minutes. The sera thus obtained were measured for GPT and GOT activities using a GPT and GOT kit (Asan Pharm.) and the results are summarized in Table 1, below.

**TABLE 1**

| GPT and GOT Activities | | |
|---|---|---|
| Group | GPT(IU/L) | GOT(IU/L) |
| Negative Control | 24.21±1.32 | 58.94±3.24 |
| Positive Control | 43.36±2.71 | 97.59±4.62 |
| Test Group 1 (fed with the extract of Working Example 1) | 28.92±3.13** | 65.73±2.83** |
| Test Group 2 (fed with the extract of Working Example 2) | 32.51±2.65** | 72.64±3.49** |
| Test Group 3 (fed with the extract of Working Example 3) | 35.74±2.43** | 81.82±5.27* |
| * *p*<0.05 ** *p*<0.01 | | |

### EXPERIMENTAL EXAMPLE 1-1-2: Hepatic Lipid Level

Changes in hepatic lipid level with the administration of the extracts were measured using the Zlatkis et al. method [Zlatkis A., Zak B., Anal. Biochem, 29, 143-148, 1969].

After the completion of the experiments in Experimental Example 1-1-1, rats in each group were sacrificed and the liver was excised therefrom. Saline was added in an amount of 2 mℓ per 1 g of the liver before the liver was homogenized in a homogenizer. Lipids were extracted using 3 mℓ of a mixture of chloroform and methanol (2:1), followed by centrifugation at 3000 rpm for 10 minutes. After the upper chloroform layer was harvested, chloroform was removed. Total cholesterol and triglyceride levels in the sample thus obtained were measured with the aid of commercially available kits (AM 202-K and AM 157S-K for total cholesterol and triglyceride, respectively, both from Asan Pharm.) and the results are summarized in Table 2, below.

**TABLE 2**

| Total Cholesterol and Triglyceride Levels in Hepatic Tissue | | |
|---|---|---|
| Group | Total Cholesterol (mg/dℓ) | Trigyceride (mg/dℓ) |
| Negative Control | 143.53±3.73 | 68.93±4.24 |
| Positive Control | 325.92±7.34 | 178.84±3.25 |
| Test Group 1 (fed with the extract of Working Example 1) | 243.63±2.93** | 125.37±4.26** |
| Test Group 2 (fed with the extract of Working Example 2) | 282.95±4.47** | 156.53±5.65** |
| Test Group 3 (fed with the extract of Working Example 3) | 295.84±5.25** | 164.72±3.36* |
| * *p*<0.05, ** *p*<0.01 | | |

### EXPERIMENTAL EXAMPLE 1-2: Improvement of Alcohol-Induced Fatty Liver

### EXPERIMENTAL EXAMPLE 1-2-1: Measurement of GPT and GOT Activity

Test animals were acclimated as in Experimental Example 1-1-1 and fed normally for two weeks. They were divided into five groups of five. Of the five groups, one was used as a negative control (normal) fed with nether alcohol nor the extracts of the Working Examples, another one as a positive control orally administered with 20% alcohol (15 mg/kg/day), and the other three groups as test groups orally administered with 20% alcohol (15 mg/kg/day) plus the extracts of the Working Examples. Diet used for the animals were AIN-76 food (Feedlab). The feeding continued for two weeks.

GPT and GOT activities in the animals were measured in the same manner as in Experimental Example 1-1-1 and the results are summarized in Table 3, below.

**TABLE 3**

| GPT and GOT Activities | | |
|---|---|---|
| Group | GPT(IU/L) | GOT(IU/L) |
| Negative Control | 23.17±2.42 | 60.47±4.28 |
| Positive Control | 46.82±3.54 | 112.52±3.47 |
| Test Group 1 (fed with the extract of Working Example 1) | 29.63±4.27** | 72.54±3.36** |
| Test Group 2 (fed with the extract of Working Example 2) | 38.31±4.89* | 82.37±2.69** |
| Test Group 3 (fed with the extract of Working Example 3) | 41.62±1.62* | 88.61±4.85** |
| * *p*<0.05 ** *p*<0.01 | | |

### EXPERIMENTAL EXAMPLE 1-2-2: Hepatic Lipid Level

After the completion of the experiments in Experimental Example 1-2-1, the animals in each group were sacrificed and the liver was excised therefrom. Hepatic lipid levels were measured in the same manner as in Experimental Example 1-1-2. The results are given in Table 4, below.

**TABLE 4**

| Total Cholesterol and Triglyceride Levels in Hepatic Tissue | | |
|---|---|---|
| Group | Total Cholesterol (mg/dℓ) | Triglyceride (mg/dℓ) |
| Negative Control | 132.72.±4.75 | 65.64±3.21 |
| Positive Control | 335.24.±5.63 | 182.92±4.83 |
| Test Group 1 (fed with the extract of Working Example 1) | 264.74±3.82** | 138.47±5.50* * |
| Test Group 2 (fed with the extract of Working Example 2) | 284.58±5.37** | 159.04±5.33* * |
| Test Group 3 (fed with the extract of Working Example 3) | 287.64±4.08** | 165.54±3.92* * |
| * *p*<0.05, ** *p*<0.01 | | |

### EXPERIMENTAL EXAMPLE 2 : Assay for Activity of Mitigating Hangover

To examine whether the extracts of the Examples can mitigate hangover, changes in alcohol dehydrogenase (ADH) and aldehyde dehydrogenase (ALDH) activities were monitored.

ADH and ALDH activities were determined using a modification of the Bostian et al. method [Bostian KA, et al. Biochem. J. 173:787-798(1978)].

First, an ADH and an ALDH fraction were obtained from an S9 rat live homogenate (Moltox Co., Boone, Nc, USA). A reaction mixture for ADH activity assay consisted of 0.05 mg of each of the extracts of Examples (or plus the extract of Reference Examples), 1.4 mℓ of distilled water, 0.75 mℓ of 1M Tris-HCl (pH 8.8), 0.3 mℓ of 20mM NAD+, 0.3 mℓ of ethanol and 0.15 mℓ of the ADH fraction while a reaction mixture for ALDH activity assay comprised 0.5 mg of each of the extracts of Examples (or plus the extract of Reference Examples), 2.1 mℓ of distilled water, 0.3 mℓ of 1M Tris-HCl (pH 8.8), 0.1 mℓ of 20mM NAD+, 0.1 mℓ of 3M KCl, 0.1 mℓ of 0.33 M 2-mercaptoethanol and 0.1 mℓ of the ALDH fraction.

The activity was determined by measuring the conversion from NAD+ to NADH with absorbance at 340 nm after reaction at 30°C for 5 minutes. The results are summarized in Table 5, below.

**TABLE 5**

| | | |
|---|---|---|
| ADH and ALDH Activities | | |

| Group | Total Cholesterol (mg/dℓ) | Triglyceride (mg/dℓ) |
|---|---|---|
| Negative Control | 1.0±0.13 | 1.0±0.11 |
| Test Group 1 (fed with the extract of Example 1) | 1.97±0.14** | 2.04±0.28** |
| Test Group 2 (fed with the extract of Example 2) | 1.63±0.24** | 1.58±0.16** |
| Test Group 3 (fed with the extract of Example 3) | 1.59±0.05** | 1.61±0.19** |
| Test Group 4 (fed with a mixture) | 2.54±0.14** | 2.75±0.22** |
| Test Group 4 was fed with a mixture of the extract of Working Example 1 and two extracts of Reference Examples (The quantity of the extract of Working Example 1 : that of the extract of Reference Example 1 : that of the extract of Reference Example 2 = 1: 1:1). | | |
| * *p*<0.05, ** *p*<0.01 | | |

### EXPERIMENTAL EXAMPLE 3: Assay for Activity of Suppressing Obesity

After the breeding in Experimental Example 1-1-1 was completed, changes in the body weight of the rats were measured (body weight after breeding minus body weight before breeding) and the results are represented as MEAN±SD values and summarized in Table 6, below.

**TABLE 6**

| Weight Change (Unit : g) | |
|---|---|
| Group | Change in Body Weight |
| Negative Control | 127.07±12.34 |
| Positive Control | 159.54±15.86 |
| Test Group 1 (fed with the extract of Working Example 1) | 95.84±13.02** |
| Test Group 2 (fed with the extract of Working Example 2) | 111.92±9.32* |
| Test Group 3 (fed with the extract ofWorking Example 3) | 108.52±13.40* |
| * *p*<0.05, ** *p*<0.01 | |

### Statistics

Results of all experiments were expressed as Mean±SD values. The results were evaluated using ANOVA and Duncan multiple test.

### Sequence List Free Text

None

## Claims

1. A composition for improving fatty liver, including at least one selected from the group consisting of a whole tomato plant extract, a Torilis japonica decandolle extract and a Lithospermum erythrorhizon Siebold & Zucc extract as an active ingredient.

2. The composition of claim 1, wherein the whole tomato plant extract is prepared by subjecting tomato leaves, stems, flowers, roots or a combination thereof to extraction with a solvent selected from among methanol, ethanol, acetone, ethyl acetate, saturated normal-butanol, chloroform, methylene chloride, water and a combination thereof.

3. The composition of claim 1, wherein the whole tomato plant extract is prepared by subjecting tomato leaves, stems, flowers, roots or a combination thereof to extraction with a solvent selected from among ethanol, water and a combination thereof.

4. The composition of claim 1, wherein the Torilis japonica decandolle extract is prepared by subjecting Torilis japonica decandolle leaves, stems, flowers, roots or a combination thereof to extraction with a solvent selected from among methanol, ethanol, acetone, ethyl acetate, saturated normal-butanol, chloroform, methylene chloride, water and a combination thereof.

5. The composition of claim 1, wherein the Torilis japonica decandolle extract is prepared by subjecting Torilis japonica decandolle leaves, stems, flowers, roots or a combination thereof to extraction with a solvent selected from among ethanol, water and a combination thereof.

6. The composition of claim 1, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among methanol, ethanol, acetone, ethyl acetate, saturated normal-butanol, chloroform, methylene chloride, water and a combination thereof.

7. The composition of claim 1, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof.

8. The composition of any one of claims 1 to 7, wherein the composition is a food composition.

9. The composition of claim 8, wherein the food composition is a beverage.

10. The composition of any one of claims 1 to 7, wherein the composition is a pharmaceutical composition.

11. A food composition for preventing a disease associated with fatty liver, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof;
the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof; and
the disease associated with fatty liver is hepatitis, hepatocirrhosis or liver cancer, said food composition being for a beverage.

12. A pharmaceutical composition for preventing a disease associated with fatty liver, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof;
the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof; and
the disease associated with fatty liver is hepatitis, hepatocirrhosis or liver cancer.

13. A composition for mitigating hangover, including at least one selected from the group consisting of a whole tomato plant extract, a Torilis japonica decandolle extract and a Lithospermum erythrorhizon Siebold & Zucc extract as an active ingredient.

14. The composition of claim 13, wherein the whole tomato plant extract is prepared by subjecting tomato leaves, stems, flowers, roots or a combination thereof to extraction with a solvent selected from among methanol, ethanol, acetone, ethyl acetate, saturated normal-butanol, chloroform, methylene chloride, water and a combination thereof.

15. The composition of claim 13, wherein the whole tomato plant extract is prepared by subjecting tomato leaves, stems, flowers, roots or a combination thereof to extraction with a solvent selected from among ethanol, water and a combination thereof.

16. The composition of claim 13, wherein the Torilis japonica decandolle extract is prepared by subjecting Torilis japonica decandolle leaves, stems, flowers, roots or a combination thereof to extraction with a solvent selected from among methanol, ethanol, acetone, ethyl acetate, saturated normal-butanol, chloroform, methylene chloride, water and a combination thereof.

17. The composition of claim 13, wherein the Torilis japonica decandolle extract is prepared by subjecting Torilis japonica decandolle leaves, stems, flowers, roots or a combination thereof to extraction with a solvent selected from among ethanol, water and a combination thereof.

18. The composition of claim 13, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among methanol, ethanol, acetone, ethyl acetate, saturated normal-butanol, chloroform, methylene chloride, water and a combination thereof.

19. The composition of claim 13, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof.

20. The composition of claim 13, including the whole tomato plant extract plus an Alnus japonica Steud extract and a Sorbus commixta extract, said Alnus japonica Steud extract being prepared by subjecting Alnus japonica Steud leaves, stems, flowers, roots or a combination thereof to extraction with a solvent selected from among ethanol, water and a combination thereof, said Sorbus commixta extract being prepared by subjecting Sorbus commixta leaves, stems, flowers, roots or a combination thereof to extraction with a solvent selected from among ethanol, water and a combination thereof.

21. The composition of any one of claims 13 to 20, wherein the composition is a food composition.

22. The composition of claim 21, wherein the food composition is a beverage.

23. The composition of any one of claims 13 to 20, wherein the composition is a pharmaceutical composition.

24. A composition for improving obesity, including at least one selected from the group consisting of a whole tomato plant extract, a Torilis japonica decandolle extract and a Lithospermum erythrorhizon Siebold & Zucc extract as an active ingredient.

25. The composition of claim 24, wherein the whole tomato plant extract is prepared by subjecting tomato leaves, stems, flowers, roots or a combination thereof to extraction with a solvent selected from among methanol, ethanol, acetone, ethyl acetate, saturated normal-butanol, chloroform, methylene chloride, water and a combination thereof.

26. The composition of claim 24, wherein the whole tomato plant extract is prepared by subjecting tomato leaves, stems, flowers, roots or a combination thereof to extraction with a solvent selected from among ethanol, water and a combination thereof.

27. The composition of claim 24, wherein the Torilis japonica decandolle extract is prepared by subjecting Torilis japonica decandolle leaves, stems, flowers, roots or a combination thereof to extraction with a solvent selected from among methanol, ethanol, acetone, ethyl acetate, saturated normal-butanol, chloroform, methylene chloride, water and a combination thereof.

28. The composition of claim 24, wherein the Torilis japonica decandolle extract is prepared by subjecting Torilis japonica decandolle leaves, stems, flowers, roots or a combination thereof to extraction with a solvent selected from among ethanol, water and a combination thereof.

29. The composition of claim 24, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among methanol, ethanol, acetone, ethyl acetate, saturated normal-butanol, chloroform, methylene chloride, water and a combination thereof.

30. The composition of claim 24, wherein the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof.

31. The composition of any one of claims 24 to 30, wherein the composition is a food composition.

32. The composition of claim 31, wherein the food composition is a beverage.

33. The composition of any one of claims 24 to 30, wherein the composition is a pharmaceutical composition.

34. A food composition for preventing a disease associated with obesity, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof;
the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof; and
the disease associated with obesity is diabetes or hypertension, said food composition being for a beverage.

35. A pharmaceutical composition for preventing a disease associated with obesity, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof;
the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof; and
the disease associated with obesity is diabetes or hypertension.

36. A food composition for dieting, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof; and
the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof

37. The food composition of claim 36, wherein the composition is a beverage.

38. A pharmaceutical composition for dieting, including at least one selected from the group consisting of a whole tomato plant extract, a Torilis japonica decandolle extract and a Lithospermum erythrorhizon Siebold & Zucc extract as an active ingredient, wherein:
the whole tomato plant extract is prepared by subjecting tomato leaves, stems, flowers, roots or a combination thereof to extraction with a solvent selected from among ethanol, water and a combination thereof;
the Torilis japonica decandolle extract is prepared by subjecting Torilis japonica decandolle leaves, stems, flowers, roots or a combination thereof to extraction with a solvent selected from among ethanol, water and a combination thereof; and the Lithospermum erythrorhizon Siebold & Zucc extract is prepared by subjecting Lithospermum erythrorhizon Siebold & Zucc leaves, stems, flowers, roots or a combination thereof to extraction with a solvent selected from among ethanol, water and a combination thereof.
